# EUROPEAN PATENT SPECIFICATION

(11) **EP 3 302 337 B1**
(45) Date of publication and mention of the grant of the patent: **02.02.2022**
(21) Application number: 16742392.0
(22) Date of filing: 03.06.2016
(51) Int. Cl.: A61B 50/36, A61L 11/00, B02C 19/00, A61L 2/08, A61L 2/18, A61L 2/24, A61L 2/20, B09B 3/00

(54) **REMOTELY-CONTROLLED IN-SITU TREATMENT OF INFECTIOUS HEALTHCARE WASTE PRODUCED BY SMALL-MEDIUM WASTE PRODUCERS**
FERNGESTEUERTE IN-SITU-BEHANDLUNG VON INFEKTIÖSEM MEDIZINISCHEM ABFALL VON KLEINEN BIS MITTLEREN ABFALLERZEUGERN
TRAITEMENT IN SITU COMMANDÉ À DISTANCE DE DÉCHETS MÉDICAUX INFECTIEUX PRODUITS PAR DES PETITS À MOYENS PRODUCTEURS DE DÉCHETS

(30) Priority: 03.06.2015 EP 15170604
(43) Date of publication of application: 11.04.2018
(73) Proprietor: RE3CUBE S.r.l., 10126 Torino (IT)
(72) Inventor: SCIBILIA, Luciano, 10126 Torino (IT); LACROCE, Renato, 10126 Torino (IT); BERT, Alberto, 10126 Torino (IT); BECCHIO, Marco, 10126 Torino (IT)
(74) Representative: Burchielli, Riccardo
(86) International application number: PCT/IB2016/053274
(87) International publication number: WO 2016/193944

(56) References cited:
- WO-A2-2007/111918
- FR-A1- 2 733 153
- US-A1- 2009 123 339
- US-A1- 2013 175 373
- US-B2- 8 195 328

## Description

### Technical Field of the Invention

The present invention relates to remotely-controlled *in-situ* treatment of infectious healthcare waste produced by small-medium waste producers.

### State of art

As known, healthcare waste is produced by facilities that carry out medical and veterinary activities of prevention, diagnosis, treatment, rehabilitation and research. The portion of healthcare waste defined as "infectious" includes waste from infectious isolation environments, disposable medical materials or contaminated veterinary biological fluids, sharp and prickly waste, etc.

Infectious healthcare waste is subject to specific national and international regulations that regulate its management, and in particular handling, storage, packaging, transportation, labelling, traceability and disposal. In particular, regulations prohibit mixing infectious healthcare waste with other hazardous or urban waste and damping, and prescribe waste treatment as close as possible to the production site, to minimize handling and the consequent risk to public health. The disposal is expected to happen by incineration in suitable plants or by sterilization/sanitization. Since the geographically capillary presence of suitable incineration plants is rare, sterilization/sanitization is considered a preferable alternative.

The main producers of infectious healthcare waste are large hospitals: there are however a number of other small producers of infectious healthcare waste such as dentists, veterinary surgeons, general practitioners, medical specialists, small to medium clinics, beauticians, tattoo artists, etc. Management of infectious healthcare waste for these small producers typically requires periodic collection of the waste and its transportation to authorized storage centres, by authorized personnel with suitable vehicles, the consolidation of loads and finally sending to disposal (via larger vehicles) at medium and large plants. The limited per capita waste production, the geographically capillary spread of small producers, the difficulty in obtaining the necessary permits, and the strong use of logistics make the current management system burdensome, anti-ecological and dangerous.

US 2013/175373 A1 discloses an integrated medical waste management and treatment system that may include sensors, interlocks, communications links and/or other features for determining if the waste itself, the decontaminating disinfectant used in the process, or the status of the system are consistent with recommended or authorized system operation. System operation may be terminated if a condition inconsistent with recommended or authorized system operation is detected. Such compliance apparatus may include an electronic scale for determining the weight of the waste loaded into the receiver compartment, a metal detector, or a sensor for determining if the decontaminating disinfectant is a recommended or authorized disinfectant. A communications link may be provided one or more systems to transmit information to a central station to deliver updates or commands associated with the recommended or authorized operation of each system.

US 2009/123339 A1 discloses bio-waste sterilizers with waste transport vessels that can be intermittently heated and pressurized, for example, with steam, and are interconnected to provide multiple sterilization pathways for waste as the waste is transported. Selected sterilization pathways may depend on attributes of the waste. Sterilization factors, such as mechanical shredding, application of steam, application of pressure, maintenance of temperature, separation of liquid, etc., are integrated into the waste transport features of the system, which can be made compact to save space while processing relatively large bulk amounts of materials, such as medical wastes. The system maintains safe isolation of biohazardous components in the waste from the external environment. A system controller provides parameters management, smart sterilization cycle control, quality assurance, safety management, diagnostics, and reporting over a network.

WO 2007/111918 A2 discloses systems and methods for collection and disposal of infectious and medical waste, including a system with a body having a chamber that receives a container of medical waste. The chamber may include a canister that has limited access to the interior of the canister for safe collection of sharps material. The chamber may have at least one plate heater coupled thereto for providing heat to the chamber and a plurality of fins on the chamber to assist in cooling the chamber.

US 8 195 328 B2 discloses a system and method related to dispensing and disposing medical items. The dispensing portion is generally configured to dispense medical items stored within compartments based on dispensing instructions. The disposal portion is generally configured to sort waste items into a plurality of containers according to applicable rules and regulations governing the handling and/or disposal of such items. In some embodiments, a system comprises sorting stations each of which houses a number of disposable containers. Each station can identify an item of waste, determine the most appropriate container for the item, and facilitate disposal of the item in the appropriate container.

FR 2 733 153 A1 discloses a portable or mobile container for collecting and sterilising hazardous waste, comprising a stainless steel chamber for storing waste, sealed openings for introducing waste, a compartment for an ozone or ethylene oxide cartridge or an ozone generator connected to the storage chamber and means for emptying waste from the chamber. The sealed opening of the container is pref. equipped with a cellular distributor with a ratchet mechanism which allows it to turn in one direction only, making it impossible to retrieve any article once placed in the container. The process has detectors which determine how much volume remains. The storage chamber has a removable base.

### Object and Summary of the Invention

The Applicant came to the consideration that the lack of solutions dedicated to the market sector of small producers of infectious healthcare waste can essentially be explained by considering that the main technologies developed for waste sterilization tend to be complex and costly, both in their production (each machine is a prototype) and in their operation, which is bound to their installation site, and they become economically viable only for medium to large waste flow.

Consequently, the Applicant came to the consideration that an ideal management of infectious healthcare waste from small producers should regularly sterilize waste at the producer site. However, current sterilization systems for infectious healthcare waste use technologies having features (management complexity, risk, size, power consumption, etc.) that make their use by unskilled operators and in a domestic urban context difficult, dangerous or uneconomic.

The purpose of the present invention is therefore to introduce a concept of simplicity and reduction of production and operating costs, allowing to scale down to small volumes, retaining efficiency, thus making *in-situ* sterilization practical and economical even for small waste productions.

More precisely, the purpose of the present invention is to provide a solution for the management of infectious healthcare waste dedicated to small-medium waste producers, architecturally simple, inexpensive and easy to install and use. The solution should permit to address the need to simplify and reduce user undertakings, costs, risks and waste disposal impact, by treating and sterilizing healthcare waste directly at the time and site of production.

According to the present invention, a remotely-controlled system for *in-situ* treatment of infectious healthcare waste produced by small-medium waste producers is provided, as claimed in claim 1. Further embodiments of the invention are defined by the dependent claims.

### Brief Description of Drawings

Figure 1 schematically illustrates the architecture of a remotely-controlled system for *in-situ* treatment of infectious healthcare waste produced by small-medium waste producers according to the present invention;
Figures 2 and 3 show, in longitudinal section, floor-mounting and wall-mounting versions of an infectious healthcare waste collection and treatment device intended to be installed at small-medium waste producers' premises;
Figure 4 illustrates a block diagram of the infectious healthcare waste collection and treatment device shown in Figures 2 and 3;
Figure 5 illustrates a waste input device which is part of the infectious healthcare waste collection and treatment device shown in Figures 2 and 3;
Figure 6 illustrates a filtering unit which is part of a depressurization and vents treatment device which is in turn part of the infectious healthcare waste collection and treatment device shown in Figures 2 and 3;
Figure 7 illustrates a block diagram of an electronic control and communication device which is part of the infectious healthcare waste collection and treatment device illustrated in Figures 2 and 3;
Figures 8 and 9 illustrate cross- and longitudinal sections, respectively, of a different embodiment of the floor-mounting version of the infectious healthcare waste collection and treatment device intended to be installed at small-medium waste producers' premises;
The Figures 10 and 11 illustrate cross- and longitudinal sections, respectively, of a different embodiment of the wall-mounting version of the infectious healthcare waste collection and treatment device intended to be installed at small-medium waste producers' premises;
Figures 12 and 13 illustrate an waste emptying and storage device which is part of the infectious healthcare waste collection and treatment device illustrated in Figures 8-11; and
Figure 14 illustrates a sterilization effectiveness verification device which is part of the infectious healthcare waste collection and treatment device illustrated in Figures 8-11.

### Detailed Description of the Preferred Implementation Forms of the Invention

The present invention will now be described in detail, with reference to the attached figures, to enable a skilled person to implement and use it. Various modifications to the described embodiments will be immediately apparent to the expert and the generic principles described may be applied to other embodiments and applications, without thereby exiting from the protective scope of the present invention, as defined in the appended claims. Therefore, the present invention should not be considered limited to the forms of embodiment described and illustrated, but should be granted with the widest scope compliant with the described and claimed principles and features .

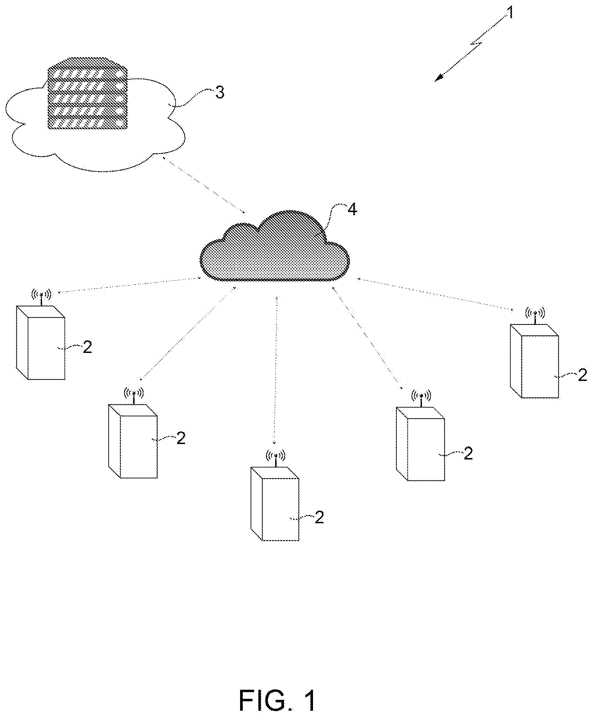

With reference to Figure 1, reference numeral 1 schematically designates, as a whole, a remotely-controlled system to *in-situ* treat infectious healthcare waste produced by small-medium waste producers, according to the present invention.

The *in-situ* treatment system 1 comprises essentially:
- infectious healthcare waste collection and treatment devices 2 intended to be installed at small-medium waste producers' premises to collect and treat infectious healthcare waste and designed to be compliant with use in venues intended for residential purposes, practicing a profession or providing a service, such as domestic venues, offices, medical, dental and veterinary practices, clinics, or the like, and
- a remote control centre 3 in communication with the infectious healthcare waste collection and treatment devices 2 via a long-range communication system 4, conveniently a packet-switching communication network, such as the Internet, or a cellular network, for example 4G / GPRS.

As will be described in greater detail hereinafter, the infectious healthcare waste collection and treatment devices 2, hereinafter referred to as "robots" for the sake of brevity, are provided with a sensory system to monitor their own operation and are in communication with the remote control centre 3, which is configured to receive and process data provided by the robots 2 and to monitor, control and report on the robot operation based on the specific applications and/or users' needs, in particular to start waste treatment cycles according to configurations set by the waste produces, for example every night, at a predefined time, or at the occurrence of certain events, or based on data provided by the robots, for example as a function of the robots filling level, or based on a combination of configuration parameters and data supplied by the robots 2.

A waste treatment cycle comprise essentially waste shredding, to homogenize and reduce waste volume, followed by sterilization. At the end of each waste treatment cycle, the contents of the robot has been transformed from dangerous infectious healthcare waste in a sterile fluff waste classifiable as a special non-hazardous waste and/or as unsorted urban waste. This product can be conveniently used as a means to generate energy to replace traditional fossil fuels in plants such as incinerators, cement plants, steel plants, thermal power plants, etc., even after pelletizzation. Furthermore, thanks to its high calorific value due to the high plastic and cellulose content, the sterilized healthcare waste is of particular interest for Secondary Solid Fuel plants. For ease of management, and considered the small quantities at stake for each small waste producer, such waste may also be assimilated to urban waste and collected and disposed of through the same chain.

The remote control centre 3 is configured to produce detailed reports of waste treatment cycles that are provided to waste producers, for example as attachments to an email, or as a file downloaded from a dedicated website. Reports have all needed features to meet regulatory requirements on waste treatment and therefore relieve waste producers from criminal responsibility and from the bureaucratic fulfilments associated with the current micro-collection management system.

At a frequency dictated by regulatory requirements and the filling level of the robots, the remote control centre 3 is also configured to schedule the collection of the sterilized/sanitized waste and issues waste recovery orders.

Compared to a conventional autoclave, the robots sterilize waste and collect internally the crushed, homogenized and sterilized waste. The sterilized waste can be extracted from a robot in various ways, a non-exhaustive list is reported below:
- by transferring it into an easily replaceable closed collection container provided within the same robot, where sterilized/sanitized waste is compacted. This allows to use a small sterilization volume sized on the daily production of infectious waste, which volume at the end of the treatment cycle is clean and sterile; the waste treatment cycles can therefore be repeated several times without the need to act on the robot;
- by recovering the entire robot followed by a maintenance phase, which is carried out at a specialized site also managing robot collection;
- by replacing part of the robot, in particular the waste collection container, to limit the transported weight and the chances of breakage of the more delicate components, such as electronics and engines; the opening of the waste collection container is carried out at a specialized site also managing the collection of the removed parts of the robot; or
- by emptying the waste treatment tank, for example with a HEPA filter vacuum cleaner, which is transported in turn, after several cleanings, at a specialized site managing the collection of such waste and therefore its disposal or pelletizzation, with the advantage of not moving any of the robot components.

The benefits resulting from the management process described above can be summarized as: management simplicity, safety, reduction of waste handling operations, especially for infectious waste, accumulation of sterile waste rather than infectious waste, decrease of environmental and energy impact of transport activities, reduction of risks arising from the transport of hazardous waste.

The robots are shredding and sterilization/sanitization devices. Testing, commissioning and validation operations are performed by the manufacturer prior to delivering the robots, all the documentation is always attributable to robots through unique serial numbers. Only infectious healthcare waste is placed in the robot as they are produced, and are regularly subject to waste treatment cycles. At the end of each waste treatment cycle, the robots are opened again to allow the introduction of new waste for a subsequent treatment. With periodicity dictated by the current regulations (for example, every 100 cycles or every 3 months), the system is arranged to perform a verification of the effectiveness of the waste sterilization cycle.

The latter operation is performed by means of microbiological tests commonly used in healthcare facilities, and can be performed as part of the service offered to the waste producers or by the waste producers themselves, reducing service costs. In order to facilitate such operation for waste producers, the robots are provided with appropriate cells and the remote control centre reminds waste producers the need to perform the verification, guides them during execution, and records the outcome.

Each robot may be equipped with an integrated emptying module that, at the end of each waste treatment cycle, transfers the treated waste, through a drain valve and with the aid of a depressurization system, to a waste collection container, for example a special bag, easy to replace by the waste producer, when it is full. The emptying module can be provided with a suitable filling sensor to communicate to the remote control centre and to the waste producer the need to replace a full treated waste container with an empty one. The treated waste container may be either disposed with urban waste or stored, even for long periods in places that need no special requirements (e.g. up to 1 year), waiting for the withdrawal by waste collection operators. Thanks to the shredding operated by the robot, the volume of treated waste is equal to about 10-20% of the infectious waste from which it derives.

In contrast, if the integrated emptying module is missing, robot emptying by waste can take place at an authorized service centre or directly where the robot is located. A specialized operator works through a HEPA filter vacuum cleaner keyed with the load valve and through this extracts the treated waste, no dust of any kind is emitted in the surrounding environment and no waste could come into contact with the surrounding environment during emptying. At the end of the emptying process, the robot is again ready to use. In order to further simplify the replacement operations, in the case of withdrawal of the waste treating tank and its content, and to reduce the chances of damaging the most delicate system parts during transport, the robot is designed so that it is possible to leave a module always installed at the waste producer's site.

Given the peculiar characteristics of the robot, in case the periodic sterilization effectiveness verification is performed by the waste producer, in normal operating conditions, the need for intervention at the waste producer's site can be reduced to the sole maintenance operations or, for example, once a year, for the withdrawal of sterilized waste containers or for the robot emptying, thereby significantly reducing the service costs. The simplicity of the service, waste traceability management for each user, the waste daily treatment, the control of each pre- and post-treatment stage through the remote control centre, the certification daily transmission, etc., are a significant added value for waste producers and represent an advanced logistics concept that the system according to the invention allows to realize.

The robot looks like a small design furniture element in which the waste producer enters the waste right when is produced, just like it is done with the container currently used.

The robot is a simple machine consisting of small number of primary and secondary components of control and safety: all components are standard and are made of easily available materials.

The robot is designed to be installed very easily at the waste producer's site, just like a household (modular) appliance, it is easy to carry, and can be conveniently equipped with anti-vibration rubber supports allowing to place it on the floor. It requires a standard electrical outlet available at households (e.g. 16A) and, in the version not equipped with autonomous connectivity via telephone SIM or WI-FI, may require a wired connection to the waste producer's network. An alternative version is equipped with anti-shock supports to dampen vibrations, and can be fixed to the wall by means of common mechanical fastening systems, this allowing to keep the robot completely raised from the ground.

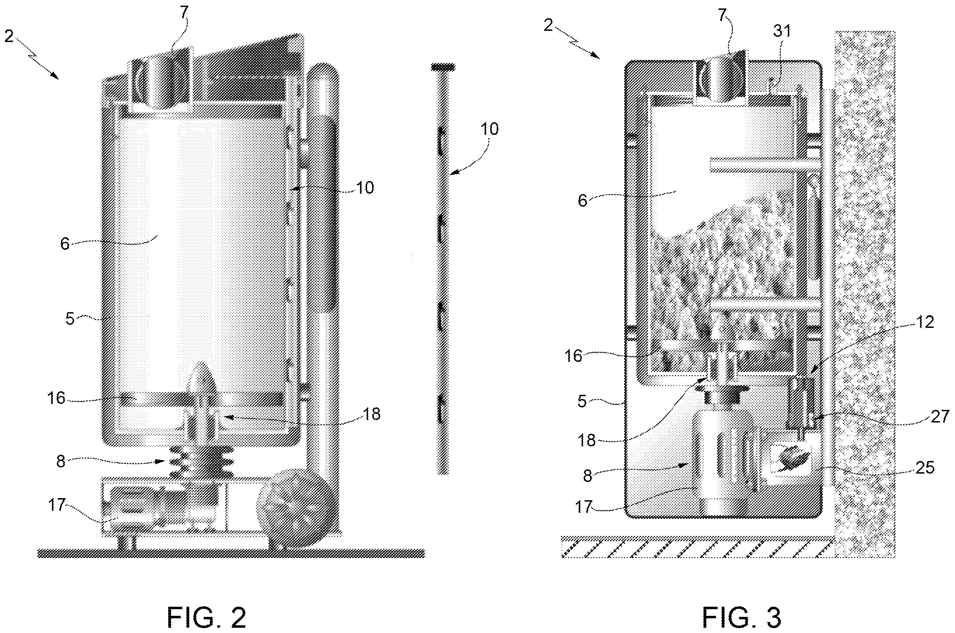

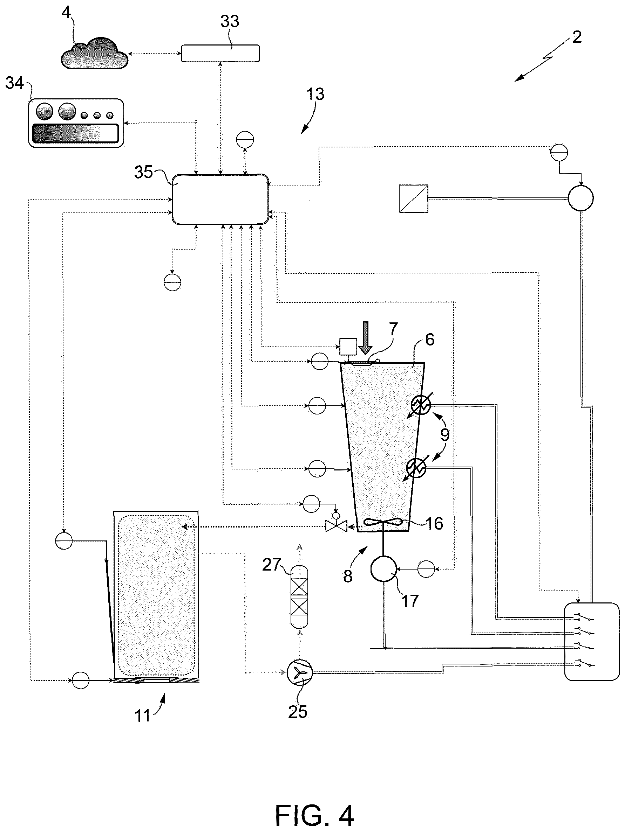

Figures 2 and 3 show two possible versions of the robot 2, a floor-mounting version and a wall-mounting version, while Figure 4 illustrates a block diagram of the robot 2.

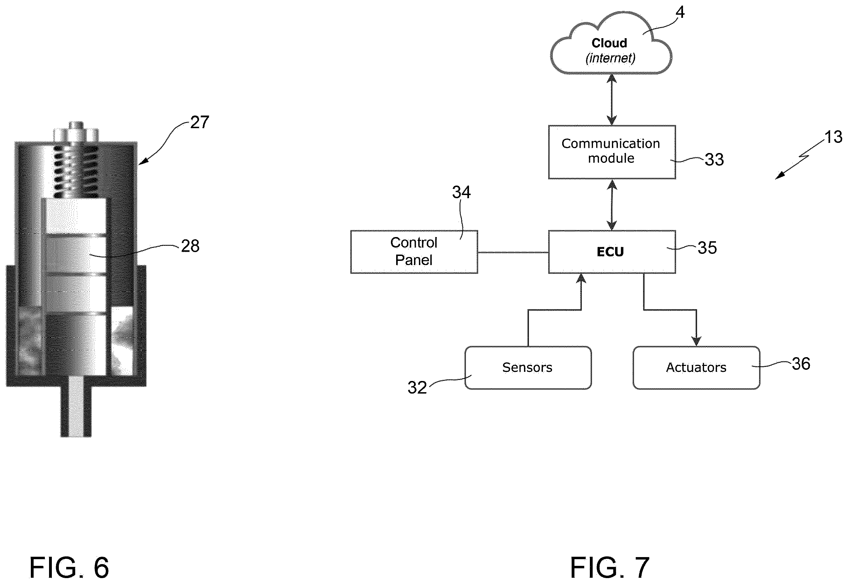

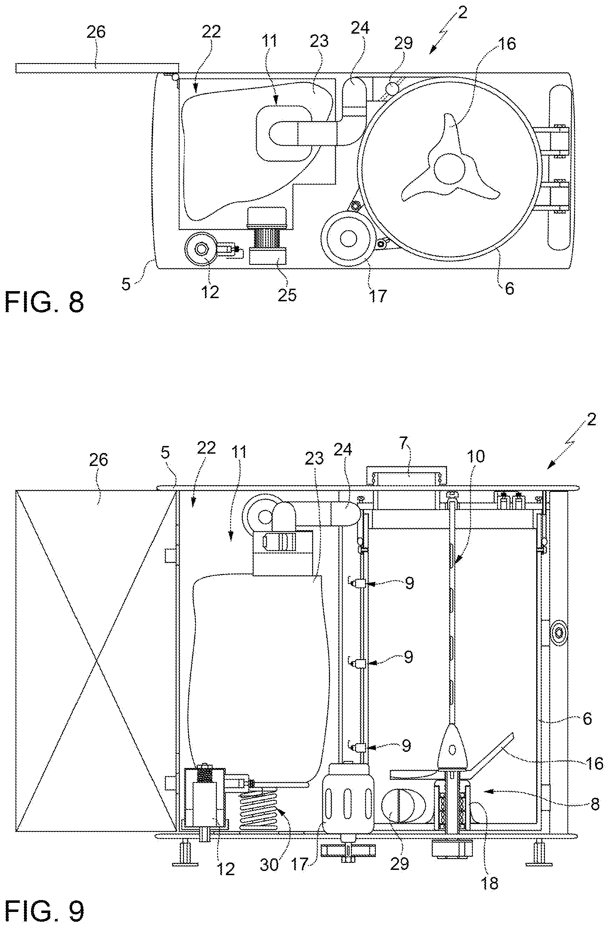

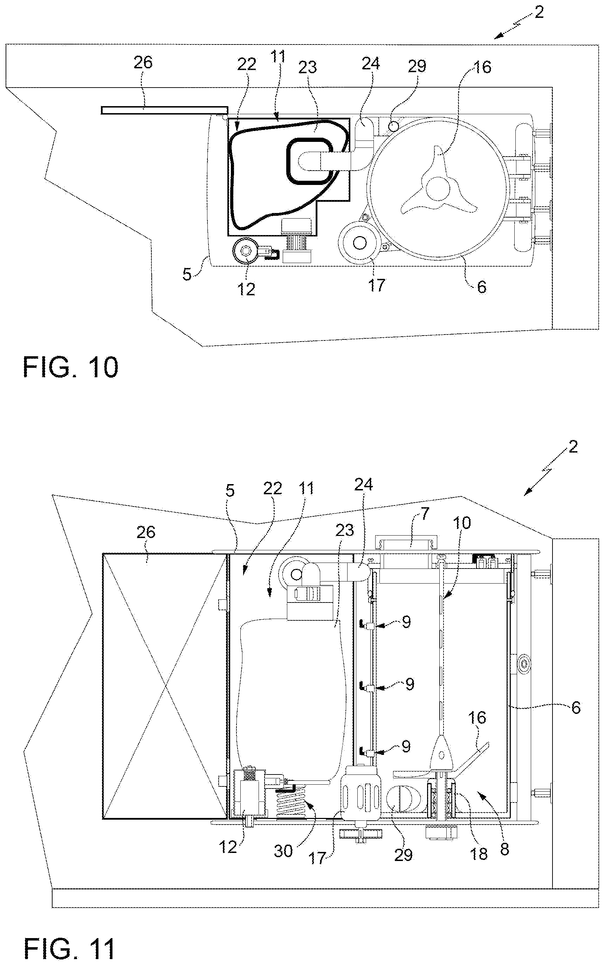

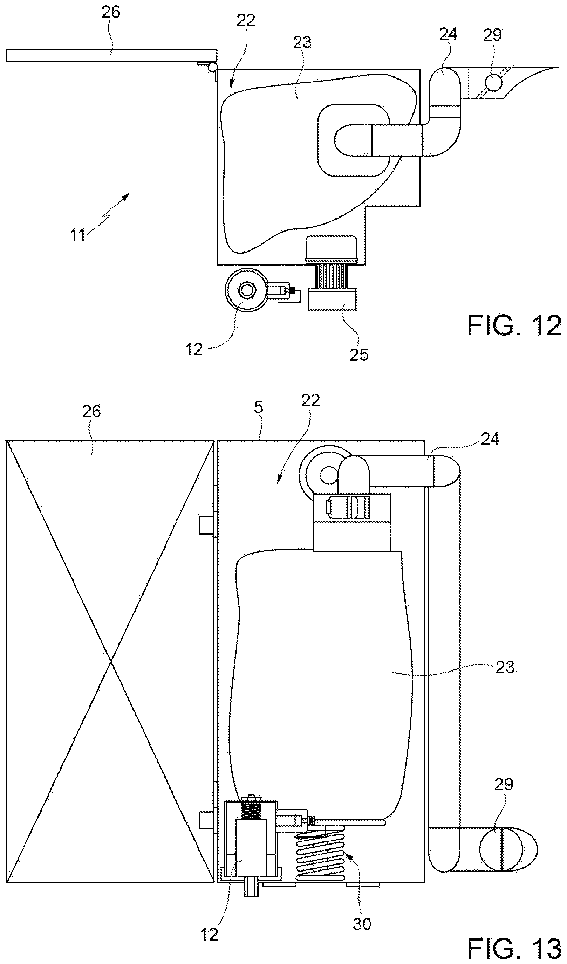

With reference to these Figures, the robot 2 essentially comprises the main functional components listed below and described individually in detail further below:
- a casing 5,
- a waste treatment tank 6 housed in the casing 5 and provided with a waste input device 7,
- a waste shredding and mixing device 8, housed partly in and partly out of the waste treatment tank 6,
- a waste sterilization device 9 (visible in Figures 9 and 11, which are relative to a different embodiment of the robot 2) associated with the waste treatment tank 6,
- a sterilization effectiveness verification device 10 removably housed in the waste treatment tank 6,
- a waste emptying and storage device 11 with sterilized waste collection (also visible in Figures 8 to 13, which are relative to a different embodiment of the robot 2) and externally coupled to the waste treatment tank 6,
- a depressurizing and air treatment device 11 externally coupled to the waste treatment tank 6, and
- an electronic control and communication device 13 (see also Figure 7) to control operation of the robot 2 and to communicate with the remote control centre 3.

### Waste treatment tank 6

The waste treatment tank is made by calendering and/or molding of stainless steel or COR-TEN steel, or electrolytically treated steel. It can have a cylindrical or any other shape, preferably generated by axial rotation. The waste treatment tank is insulated, depending on its size or on the robot body characteristics, in order to maintain the operating temperature and to avoid robot overheating, considering its use in domestic environment. The insulation also helps to contain the sound emission arising from the trituration phase.

The calendering process can be used for manufacturing the whole waste treatment tank or even for just part of it. In case the calendering of the sole peripheral wall is performed, the waste treatment tank floor and loading upper bridge can be welded or bolted to the wall.

The waste treatment tank is shaped so as to avoid formation of internal pockets where waste debris may deposit during the shredding, sterilization and emptying phases.

The loading upper bridge is provided with an inlet opening for the waste introduction and a fastening system for the waste input device.

The waste treatment tank floor is provided with a hermetically sealed coupling system for a hub transferring the motion from the external engine to an internal shredding and homogenization blade, as well as pressurization seals.

Along the perimeter wall or on the loading upper bridge and on the floor are provided threaded holes for the positioning of temperature sensors and an over-pressure safety valve.

In the robot version provided with an integrated emptying system, there is an additional drain opening, tangent to the floor, suitable for the connection to the drain valve, in order to ensure the outflow of the treated waste.

### Waste input device 7

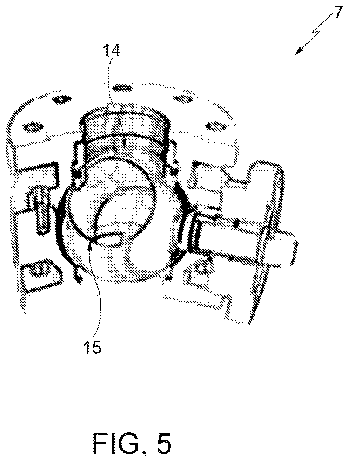

A possible embodiment of the waste input device 7 is depicted in Figure 5.

The waste input device 7 is designed to optimize usability during waste loading and possible vacuuming of the treated waste, and to ensuring maximum safety during sterilization.

The waste input device is designed to resist high temperature and high pressure and is fixed to the waste treatment tank by means of shrinking or welding or by any appropriate fixing system that guarantees in any case a suitable seal and mechanical resistance.

In particular, the waste input device 7 is configured to allow the waste treatment tank 6 to be accessible from the outside through a waste inlet 14, to allow waste to be introduced in the waste treatment tank 6, and to seal the waste inlet 14.

The waste input device 7, when in opening configuration, connects the waste treatment tank 6 with the outside of the robot 2 to allow waste introduction. When in the closing configuration, it grants the hermetic sealing and thermal and noise shielding of the waste treatment tank 6 and prevents access from the outside.

In detail, the waste input device 7 essentially comprises a hatchway or valve system 15 provided with a rotatable or translatable hatch, equipped with insulation, sealing systems, position control and locking system.

The movement of the hatch may be alternatively obtained manually, with consequent reduction of the robot manufacturing costs, or controlled by an actuator controlled in turn by the electronic control and communication device. A sensor to check the effective and complete closing of the waste treatment tank may also be provided, to allow the treatment cycle start, and a locking device operating during the treatment cycle.

The waste inlet is sized to allow an easy access for a great variety of waste, but at the same time, to avoid the entrance of waste not consistent with the intended use of the robot. The waste inlet may also have a section shaped to facilitate the waste addressing into the treatment tank during loading.

### Waste shredding and mixing device 8

The waste shredding and mixing device 8 essentially comprises a blade 16 for waste crushing and pulverizing housed on the floor of the waste treatment tank 6 and operated by an engine 17 housed outside the waste treatment tank 6 and suitably coupled to the blade 16. in particular, the blade 16 is coupled to an engine output shaft 17 through a kinetic joint 18, allowing the coupling to withstand the inertia that the solid waste or the fluff waste transmit to the system thus preserving engine life, isolating the engine thermally and from the induced currents, and finally, reducing its noise.

It is also possible to couple the blade 16 with the engine 17 through a transmission system that connects the engine output shaft to a driving hub coupled to the blade 16 by means of a system of pulleys and belts, gears, or chains.

It is also possible to provide the transmission system between the engine 17 and the blade 16 with a kinetic friction slider device capable of limiting the drive torque necessary in the starting stages of the waste shredding and mixing device 8.

The blade cutting profile geometry and its spatial orientation are inspired by the NACA profiles (particular forms of aircraft wings designed by the U.S. National Advisory Committee for Aeronautics), and are designed to fulfil the following series of functions:
dissect heterogeneous materials and with different resilience, making them slide on the cutting edge with a variable incidence as a result of its curved profile, so as to preserve the blade life and to increase its mechanical properties;
behave like a hammer acting on the ductility of the waste with hardness properties equivalent to metals, and grinding those similar to glass;
   - lift the dissected waste along a positive vertical direction parallel to the blade central axis, so as to minimize the blade body friction with the waste homogeneous mass;
   - plough the heterogeneous waste mass with the blade most peripheral rostrum, ravelling it and pressing it to the tank perimetral surface and employing the metal friction according to the laminar flows criterion;
   - mix the homogeneous mass of crushed waste to pulverize and aerate it turning it into light and homogeneous fluff;
      generate a ground effect lifting the waste from the tank plane floor, thanks to a horizontal oblong wing located close to the hub, to avoid the formation of coarse waste pockets on the tank floor; and
   - trigger a waste raising helical flow along the tank wall to homogenize the treatment temperatures during sterilization.

The blade engine may be either on/off controlled or speed-controlled. The engine does not privileges any particular technology since the motion can be generated by an internal combustion engine or by an electric motor, provided that they present the following characteristics:
the engine complies with current regulations for household appliances,
- the engine noise during the centripetal acceleration and maximum speed phases is compatible with a home environment;
the electric motor characteristics are suitable to a domestic supply in terms of: voltage, amplitude and frequency of the electric current, maximum electrical power consumption, and protection.

### Waste sterilization device 9

The shredded waste sterilization device 9 can be, for example, thermal-based and comprising an electric resistor-based heating system with heating resistors located on the peripheral wall of the waste treatment tank or, alternatively, induction-based, where heat is conveyed by convection and radiation to the waste and to the treatment tank walls. The waste mixing and homogenization action operated by the blade assure a continuous mixing of the waste being treated, that behaves as a completely mixed system. At the same time, the different heat capacities of the various materials in the waste treatment tank and originated from the introduced waste, allows an almost uniform heat distribution throughout the mass under treatment, avoiding the generation of critical areas with too low o too high temperatures. In such a system, thanks to the type of material and the flows induced by the blade, the time/temperature conditions ensuring sterilization effectiveness are typical of the processes using saturated steam under pressure. The presence of shredded material within the tank helps heath distribution and mixing motions effectiveness.

The waste sterilization device 9 and the engine 17 operating the blade 16 of the waste shredding and mixing device 8 are controlled by the electronic control and communication device 13, that identifies the start of the sterilization phase and ensures the maintenance a minimum pre-set temperature for a time sufficient to kill the microorganisms with a degree of effectiveness (Steril Assurance Level - SAL) not less than 10⁻⁶, as required by the strictest regulations.

The shredding and the production of a completely mixed waste system opens to the possible use of sterilization methods alternative to heat such as:
- chemical process: by introducing a sterilizing agent, such as sodium hypochlorite, in the waste treatment tank, it is possible to obtain sterility with no need to heat. In this case, the robot components used for temperature management are replaced by a sterilizing agent dosing system. With suitable precautions and the installation of a dedicated production module (typically with crown effect) is possible to use gaseous ozone as sterilizing agent;
- radiation process: the waste treatment tank can be exposed to ionizing or nonionizing radiation (including for example: X rays, gamma rays, microwave, laser, etc.) having penetration capacity and dosage sufficient to reach the sterility condition within the mass of material. Due to their action on the microorganisms and costs, particularly suitable for the present application are microwaves, that can replace the heating system with similar management logics, also considering that the metal of the tank acts as an effective shielding.

### Sterilization effectiveness verification device 10

Some national regulations require the periodic verification of the sterilization effectiveness by using bio-indicators appropriate to the employed sterilization process. The robot is designed to allow an easy test execution with indicator vials even by unskilled personnel and without the need for additional equipment, thus making the operation easy to perform to unskilled personnel.

The sterilization effectiveness verification device comprises essentially a verification cell 10 specifically designed to house a series of indicator vials or similar systems (such as are currently available cultural soil vials, containing the spore suspension of bacillus stearothermophilus).

The verification cell 10 is manufactured to internally ensure the same temperature conditions experienced by the waste, therefore during the process of validation and verification of the robot effectiveness, it assures the vials are subject to a legally valid sterilization verification cycle, avoiding downtime, additional costs, and need for third parties for the verification.

The verification cell 10 is always present in the robot, without vials, while the robot performs its normal functions of healthcare waste shredding and sterilization, thus being usable at any cycle to conduct verification tests on the robot operation.

It is also possible to set a phase after the verification cycle where the robot is kept closed, the blade not rotated, at a constant temperature equal to that of incubation, so as to get the vials out after 48 hours, and to read directly the result of the test. The optional use of the waste treatment tank as the incubation system is monitored by the remote control centre, that assure the maintenance of the temperature in the specific range required by the technical specifications of the used vials (typically ± 2 ° C as required by the specifications of the vials).

Technically, the verification cell can be positioned at the centre of the shredding flows, suspended axial with respect to the blade hub or arranged along the perimeter of the waste treatment tank.

The verification cell 10 is realized with a material having thermal conductivity and mechanical seal ensuring the same temperature conditions as those to which the waste is subjected, withstanding these conditions and protecting the indicator vials by mechanical actions exerted by the moving waste. Suitable materials for this purpose are stainless steel, COR-TEN steel and the traditional steel, and in general, other materials meeting the above-mentioned characteristics.

The indicator vials are introduced in the verification cell 10 inside a suitable holder allowing to always place them in a known position and recover them intact at the end of the sterilization cycle. After inserting the indicator vials in the holder, and then in the verification cell, the robot containing the material to be treated (the heat sterilization cycle can potentially be conducted with the empty tank) is started for a normal work cycle. At the end of the cycle the indicator vials can be extracted to be placed in an incubation instrument together with a control vial not subjected to the sterilization cycle (having the purpose to verify the effectiveness of the culture medium). Alternatively, the control vial can be introduced in the verification cell and the incubation performed by the robot itself, as defined by the vials usage protocol (for example 48h at 57 ° C for the vials of Bacillus stearothermophilus). The sterilization is verified, for example, in case of no colour change of the indicator vials after incubation.

The verification cell is shaped to allow the introduction of four indicator vials, in accordance with the strictest national standards. The shape of the verification cell, along with the high-speed rotation of the treated waste, allows uniform temperature conditions to be ensured in the radial direction. The particular blade conformation, as described above, generates a peripheral waste flow along helical curves that convey the waste to the centre at the top, and it falls down where it is taken up by the blade. Such motion allows to keep the temperature uniform also along the vertical direction, the axis where is most likely to observe a thermal gradient. Placing the indicator vials at different heights ensures the verification of the effectiveness of the sterilization cycle in all sites that could potentially present critical temperatures.

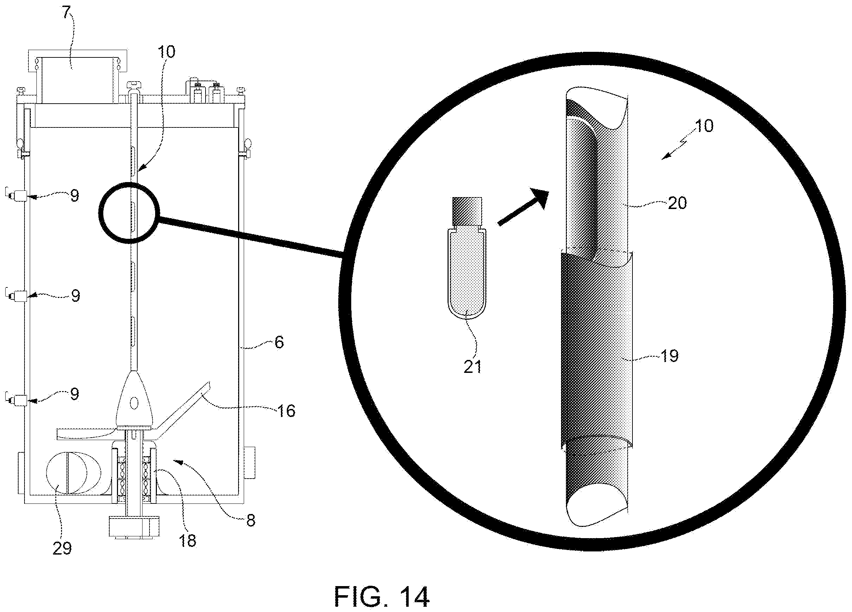

As illustrated in greater detail in Figure 14, which relates to a different embodiment of the robot 2, described later, the verification cell 10 essentially comprises a tubular casing 19, conveniently of cylindrical shape, allowing an easy introduction and extraction of the verification cell into and from the waste treatment tank 6 by unskilled operators. The verification cell further comprises a vials-holder 20 removably housed in the tubular casing 19 and having the same cross-section of the tubular casing 19, circular in the illustrated example, and a size sufficient to host the indicator vials 21 and slide freely in the verification cell. The construction material is the same of the verification cell. Appropriate openings are provided for the introduction of the indicator vials in the vials-holder, and are arranged laterally or at the top, depending on the type of adopted vials.

Thanks to this feature, the health professional may employ for the verification the same indicator vials he/she is typically already using to regularly verify the sterilization of the instruments (through an autoclave).

A possible alternative to the indicator vials are chemical sterilization indicator strips inserted in the same holder and position used for the vials, allowing sterilization efficacy verification by a colour change due to their exposure to a given temperature for a given time.

In the case sterilization systems other than heat are adopted, such as chemical agents or ionizing radiation or non-ionizing (including for example: X rays, gamma rays, microwave, laser, ...), the effectiveness test is performed using bio-indicators specific to the sterilizing agent.

### Waste emptying and storage device 11

As shown in Figures 8 to 13, which are relative to a different embodiment of the robot 2, described after, the waste emptying and storage device 11 essentially comprises a hermetically sealed compartment 22 containing a waste collection container or bag 23 of paper or other materials (typically the common vacuum cleaner bags) held by a support flange and connected to a tank-emptying opening through a transferring pipe 24. The flow from the waste treatment tank 6 to the sterilized waste collection container 23 is obtained by depressurization generated by a vacuum engine 25 fluidically communicating with the hermetically sealed compartment 22.

The waste emptying and storage device 11 is also provided with a filling monitoring device, conveniently in the form of a weighing device 30 disposed under the waste collection container 23 and connected to the electronic control and communication device 13 which, based on the measured weight, alerts the remote control centre 3 that notifies the waste producer the need to replace the waste collection container 23. Replacement of the waste collection container is done through a comfortable door 26 allowing to remove the full container 23, and connect a new and empty one to the support flange.

### Depressurizing and air filtration device 12

The waste treatment tank 6 undergoes, due to temperature and humidity possibly present in the waste, a pressure raise, above atmospheric values, that is useful to improve the performance of the robot 2 and ensure a perfect sterilization of the treated mass.

Depending on whether it is the version with or without the waste emptying and storage device 11, the following two scenarios will happen:

### Without waste emptying and storage device 11

Upon completion of each treatment cycle, the electronic control and communication device 13, in response to a command of the remote control centre 3, activates a dedicated vent valve 31 (Figure 3) located on the top of the waste treatment tank 6 to allow depressurization. Over-pressure vent pass through a filtering unit 27 shown in greater detail in Figure 6 and comprising a vent treatment cartridge 28 comprising a dust filter, possibly zeolites, active carbon, and an smell abatement system. The cartridge 28 is realized as a modular system of stacked cylindrical tablets within a compensating cylinder, around which it is possible to hold a liquid in which the output vent may bobble to ensure a further specific treatment. The tablet stack may be assembled according to specific requirements enabling multi-level vent abatements and the choice of the smell abatement system best suited for the specific treated waste and characterized by essences (aroma) chosen by the customer.

The tablet replacement is very simple thanks to the accessibility of the depressurizing valve, this operation can be performed by the user without special precautions. The neutralization of smells can also be done through the dosage of reagents directly into the treatment tank. At the end of the depressurization, the robot 2 is ready to be set to the open configuration.

### With waste emptying and storage device 11

Upon completion of each treatment cycle, the electronic control and communication device 13, in response to a command from the remote control centre 3, activates the depressurizing and air filtration device 12, which essentially comprises an emptying opening formed in the waste treatment tank 6 provided with a drain valve 29, and the vacuum engine 25, connected to the drain valve 29 through a pipe along which a filtering unit is arranged, conveniently of a cartridge type. In order to allow the vents outflow, the electronic control and communication device 13 also activates the waste input device 7 or the vent valve 31. The waste treatment tank 6 is thus depressurized and the gaseous vent possibly present is conveyed into the sterilized waste collection container 23, being thus subject to a first filtration. After the passage through the vacuum engine 25, the vents pass through a filtering unit formed by an absolute filter and, possibly, an active carbon filter to trap any organic substances and / or smell, and deodorant cartridges of the type shown in Figure 6.

The filtering unit is a modular system that can be assembled according to specific requirements enabling multi-level vent abatements and the choice of the smell abatement system best suited for the specific treated waste and characterized by essences (aroma) chosen by the customer. The tablet replacement is very simple thanks to the accessibility of the depressurizing valve, this operation can be performed by the user without special precautions.

### Electronic control and communication device 13

As shown in Figure 7, the electronic control and communication device 13 basically includes:
- a sensory system 32 comprising one or more sensors operable to measure specific physical quantities such as temperature, engine speed, fill level, volume, time, pressure, vibration, weight, etc., and to provide field data representing the measurements,
- a communication module 33 operable to communicate with the remote control centre 3 via a long-range communication system 4, conveniently a packet-switching communication network, for example the Internet, or a cellular network, for example 4G/GPRS,
- a control panel 34, and
- an electronic control unit 35 connected to the sensory system 32, to the communication module 33, to the control panel 34 and to the various actuators in the robot 2, collectively designated by reference numeral 36 and comprising the engine 17 to operate the blade 16, the vacuum engine 25 of the depressurizing and air filtration device 12, the actuator, if any, of the waste-input device 7, etc., and programmed to transmit to the remote control centre 3 field data indicative of the operation of the robot 2 and to receive from the remote control centre 3 and execute commands relating to the operation of the robot 2, and in particular commands for opening/closing the waste input device 7, activating the depressurizing and air filtration device 12 to depressurize the waste treatment tank 6, powering on/off the engine 17 to operate the blade 16, powering on/off the waste sterilization device 9, blocking operation of the robot 2 in case of emergency, etc.

Here below is an exemplary non-exhaustive list of the basic functionalities performed by the electronic control unit 35:
- starting the shredding cycle
- starting the sterilization phase
- starting the cooling phase
- reading control and / or emergency sensors
- starting the incubation phase
- managing of process anomalies
- blocking the robot in "closed position" in case of process anomalies or power outage.

In a functional variant of the robot 2 (not illustrated), in places where it is not possible to establish any type of connection to the remote control centre 3, the robot 2 can also be designed to operate independently from, and to implement the capabilities of, the remote control centre 2 (stand-alone mode).

In order to do so, the electronic control unit 35 is programmable for:
- exposing a graphical user interface to allow a user to configure and diagnose the robot 2 through an electronic device (such as a personal computer or a mobile device) externally connectable to the robot 2 through a cable or local Wi-Fi,
- starting the sterilization cycles according a configurable logic,
- internally storing data provided by the sensory system and produce and send sterilization reports (via wired connection or local Wi-Fi)
- diagnose the robot 2 and store diagnostics data accessible via an externally connectable electronic device.

### Remote control centre 3

With the installation of the system 1 according to the invention the waste producer is relieved from any task related waste management. Each physical, mechanical or administrative function is handled remotely, through the Internet and/or a 4G/GPRS cellular network, through which the remote control centre 3 is in communication with all installed robots 2.

The remote control centre 3 essentially comprises a remote server, conveniently a web server, programmed to receive and process data received from the robots 2 to manage and control all robots 2 installed at the waste producers, starting waste sterilization cycles, verifying and reporting the results. At the end of each working day of the waste producer (or according to different configuration, as indicated previously), the remote control centre 3 is configured to cause the robot 2 to perform a waste shredding and sterilization cycle. The robots 2 automatically close (or alternatively with a manual control), and start the work cycles for shredding and sterilize the introduced waste, drastically reducing its volume. Work cycles are monitored in real time by the robots 2 and analysed and documented by the remote control centre 3, that records process parameters and automatically generates treatment reports and sends them electronically to the waste producers in order to certify the waste treatments. Upon completion of the treatment cycles, the robots 2 open to allow new waste to be introduced and processed in subsequent cycles. The timing of waste treatment cycles and report features are configured from the remote control centre 3, according to a predefined protocol and based on the specific applications' and/or users' needs.

The remote control centre 3, beside monitoring and logging the sterilization cycles performed by each installed robot 2, can map their geographical location and monitor their working conditions (such as the level of utilization or filling). These features enable the remote control centre 3 to provide timely information to predict and signal the need for, and schedule, operations of sterilization efficacy verification, emptying operations, replacing vent treatment cartridges, maintenance interventions and/or robot replacement.

The remote control centre 3 also offers waste producers the possibility to access, via a web or mobile application, the treatment cycles, with relevant reports, working hours and parameters, and other information about their robot, through a dedicated web interface, accessible through authentication.

The remote control centre 3 also allows operators in charge of the maintenance of the robots 2, through a dedicated web or mobile interface accessible after authentication, the possibility to access data related to the operation and measured by sensors of a specific robot 2, for diagnostic purposes.

What previously described and illustrated with reference to Figures 1 to 6 may be subject to modifications and variations without thereby departing from the protective scope of the present invention, as defined by the appended claims.

For example, Figures 8 to 14 illustrate a different embodiment of the robot 2, which is hereinafter briefly described limited to the sole differences with respect to the embodiment illustrated in Figures 1 to 6 and using the same reference numbers to identify the same components.

In particular, the embodiment illustrated in Figures 8 to 14 differs from that shown in Figures 1 to 6 for the fact that:
- the ergonomic casing 5 has a different shape, in particular a generally parallelepiped shape,
- the filtering unit 27 is arranged downstream of the vacuum engine 26, which, in addition to transferring the sterilized waste in the collection container 23, also depressurize the waste treatment tank 6,
- the sterilization effectiveness verification device 10 is placed in axis with the blade 16 for shredding the waste, rather than laterally,
- the waste input device 11 comprises a sealing door, instead of a ball valve, the waste shredding blade 16 has a different profile, and
- the engine 17 to operate the blade 16 for waste shredding is connected to the blade 16 by means of a belt transmission.

## Claims

1. A remotely-controlled system (1) to *in-situ* treat infectious healthcare waste produced by small-medium waste producers, comprising:
- infectious healthcare waste collection and treatment devices (2) intended to be installed at small-medium waste producers' premises to collect and treat infectious healthcare waste and designed to be compliant with use in venues intended for residential purposes, practicing a profession or providing a service, such as domestic venues, offices, medical, dental and veterinary practices, clinics, or the like; wherein each infectious healthcare waste collection and treatment device (2) comprises:
- a casing (5),
- a waste treatment tank (6) housed in the casing (5) and provided with a waste input device (7) for infectious healthcare waste,
- a waste shredding and mixing device (8) housed in the casing (5) and associated with the waste treatment tank (6) to shred and mix infectious healthcare waste contained therein,
- a waste sterilization device (9) housed in the casing (5) and associated with the waste treatment tank (6) to sterilize infectious healthcare waste in the waste treatment tank (6),
- a sterilized waste collection container (23) removably housed in the casing (5) to collect sterilized waste,
- a sterilized waste emptying device (11) housed in the casing (5) to transfer sterilized waste from the waste treatment tank (6) to the sterilized waste collection container (23),
- a sensory system (32) to monitor operation of the infectious healthcare waste collection and treatment device (2); and
- an electronic control unit (35) connected to the sensory system (32), the waste shredding and mixing device (8), the waste sterilization device (9), and the waste emptying device (11), and configured to control the operation of the infectious healthcare waste collection and treatment device (2) by:
∘ starting waste treatment cycles based on the specific applications' and/or users' needs,
∘ receiving and processing waste treatment cycle-related data and generating producer-accessible reports of the performed treatment cycles.

2. The system of claim 1, wherein the electronic control unit (35) is further configured to:
∘ expose a graphical user interface to allow a user to configure and diagnose the infectious healthcare waste collection and treatment device (2) through an externally connectable electronic device, and
∘ diagnose the infectious healthcare waste collection and treatment device (2) and store diagnostics data accessible via an externally connectable electronic device.

3. The system of any one of the preceding claims, wherein the infectious healthcare waste collection and treatment devices (2) are designed to be compliant with use in venues intended for residential purposes, practicing a profession or providing a service in terms of one or more of electrical power consumption, size, noise, and electromagnetic emission.

4. The system of any one of the preceding claims, wherein the waste sterilization device (9) is designed to submit infectious healthcare waste to a dry sterilization process.

5. The system of any one of the preceding claims, wherein the waste sterilization device (9) is designed to submit infectious healthcare waste to a resistor-based or induction-based thermal sterilization process.

6. The system of any one of the preceding claims, wherein the sterilized waste collection container (23) is housed in a compartment (22) separate from the one in which the waste treatment tank (6) is housed, and the sterilized waste emptying device (11) is designed to transfer sterilized waste from the waste treatment tank (6) to the sterilized waste collection container (23) by depressurization.

7. The system of any one of the preceding claims, wherein the waste shredding and mixing device (8) essentially comprises a blade (16) to shred and pulverise waste, housed in the waste treatment tank (6) and operated by a motor (17) arranged outside the waste treatment tank (6).

8. The system of any one of the preceding claims, wherein each infectious healthcare waste collection and treatment device (2) also comprises a sterilization effectiveness verification device (10) removably housed in the waste treatment tank (6) and designed to host one or more sterilization indicators (21) so that they are subject to the same conditions present in the waste treatment tank (6).

9. The system of any one of the preceding claims, wherein each infectious healthcare waste collection and treatment device (2) further comprises a waste treatment tank depressurizing device (11) provided with an air filtration device (27).

10. The system of any one of the preceding claims, wherein the sensory system (32) is configured to measure the filling level of the sterilized waste collection container (23), and wherein the electronic control unit (35) is configured to determine and communicate to the remote control centre (3) the need/expediency to replace the sterilized waste collection container (23) with an empty one.

11. The system of any one of the preceding claims, further comprising:
- a remote control centre (3) in communication with the infectious healthcare waste collection and treatment devices (2) via a long-range communication system (4);
the electronic control units (35) of the infectious healthcare waste collection and treatment device (2) are configured to communicate with the remote control centre (3) to transmit thereto data indicating operation of the infectious healthcare waste collection and treatment devices (2) and to receive therefrom and execute commands relating to operation of the infectious healthcare waste collection and treatment devices (2);
the remote control centre (3) is configured to receive and process data from the infectious healthcare waste collection and treatment devices (2) and to configurably monitor, control and report on the operation thereof based on the specific applications' and/or users' needs.

12. The system of claim 11, wherein the remote control centre (3) is also configured to generate and send to the infectious healthcare waste collection and treatment devices (2) commands to start waste treatment cycles, based on the specific applications' and/or users' needs, in response to which commands the infectious healthcare waste collection and treatment devices (2) are configured to start corresponding waste treatment cycles; the remote control centre (3) is also configured to process waste treatment cycle-related data from the infectious healthcare waste collection and treatment devices (2) and to generate producer-accessible reports of the performed treatment cycles.

13. The system of claim 12, wherein the remote control centre (3) is also configured to monitor the operating conditions of the infectious healthcare waste collection and treatment devices (2) and to predict, signal the need for, and schedule verification/emptying/maintenance/replacement of the infectious healthcare waste collection and treatment devices (2).

14. The system of claim 12 or 13, wherein the remote control centre (3) is also configured to provide the waste producers with a Web-based or mobile application-based service of access to information concerning the waste treatment cycles performed by their infectious healthcare waste collection and treatment devices (2).

## Patentansprüche

1. Ferngesteuertes System (1) zur *lokaler* Behandlung infektiösen medizinischen Abfalls, der von kleinen-mittleren Abfallerzeugern erzeugt wird, umfassend:
- Sammel- und Behandlungsvorrichtungen (2) für infektiösen medizinischen Abfall, die angedacht sind, auf Grundstücken kleiner-mittlerer Abfallerzeuger installiert zu werden, um infektiösen medizinischen Abfall zu sammeln und behandeln, und gestaltet ist, zur Verwendung in Örtlichkeiten konform zu sein, die zu Wohnzwecken, Ausüben eines Berufs oder Bereitstellen einer Dienstleistung gedacht sind, wie private Örtlichkeiten, Büros, medizinische, zahnmedizinische und veterinärmedizinische Praxen, Kliniken oder dergleichen;
wobei jede Sammel- und Behandlungsvorrichtung (2) für infektiösen medizinischen Abfall umfasst:
- ein Gehäuse (5),
- einen Abfallbehandlungstank (6), der in dem Gehäuse (5) aufgenommen ist und mit einer Abfalleingabevorrichtung (7) für infektiösen medizinischen Abfall bereitgestellt ist,
- eine Abfallzerkleinerungs- und Mischvorrichtung (8), die in dem Gehäuse (5) aufgenommen ist und mit dem Abfallbehandlungstank (6) verknüpft ist, um darin enthaltenen, infektiösen medizinischen Abfall zu zerkleinern und vermischen,
- eine Abfallsterilisierungsvorrichtung (9), die in dem Gehäuse (5) aufgenommen ist und mit dem Abfallbehandlungstank (6) verknüpft ist, um infektiösen medizinischen Abfall in dem Abfallbehandlungstank (6) zu sterilisieren,
- einen sterilisierten Abfallsammelbehälter (23), der entnehmbar in dem Gehäuse (5) aufgenommen ist, um sterilisierten Abfall zu sammeln,
- eine sterilisierte Abfallentleerungsvorrichtung (11), die in dem Gehäuse (5) aufgenommen ist, um sterilisierten Abfall von dem Abfallbehandlungstank (6) zu dem sterilisierten Abfallsammelbehälter (23) zu befördern,
- ein Sensorsystem (32), um Betrieb Sammel- und Behandlungsvorrichtung (2) für infektiösen medizinischen Abfall zu überwachen; und
- eine elektronische Steuereinheit (35), die mit dem Sensorsystem (32), der Abfallzerkleinerungs- und Mischvorrichtung (8), der Abfallsterilisierungsvorrichtung (9) und der Abfallentleerungsvorrichtung (11) verbunden ist und konfiguriert ist, den Betrieb der Sammel- und Behandlungsvorrichtung (2) für infektiösen medizinischen Abfall zu steuern durch:
^{∘} Starten von Abfallbehandlungszyklen, basierend auf den spezifischen Anwendungen/ und/oder Bedürfnissen der Nutzer,
^{∘} Empfangen und Verarbeiten von Abfallbehandlungszyklus-bezogenen Daten und Erzeugen von erzeugerzugänglichen Berichten über die durchgeführten Behandlungszyklen.

2. System nach Anspruch 1, wobei die elektronische Steuereinheit (35) weiter für Folgendes konfiguriert ist:
^{∘} Zeigen einer grafischen Nutzerschnittstelle, um einem Nutzer zu erlauben, die Sammel- und Behandlungsvorrichtung (2) für infektiösen medizinischen Abfall durch eine extern anschließbare elektronische Vorrichtung zu konfigurieren und diagnostizieren, und
^{∘} Diagnostizieren der Sammel- und Behandlungsvorrichtung (2) für infektiösen medizinischen Abfall und Speichern von Diagnosedaten, die über eine extern anschließbare elektronische Vorrichtung zugänglich sind.

3. System nach einem der vorstehenden Ansprüche, wobei die Sammel- und Behandlungsvorrichtungen (2) für infektiösen medizinischen Abfall gestaltet sind, mit Verwendung in Örtlichkeiten, die für Wohnzwecke, Ausüben eines Berufs oder Bereitstellen einer Dienstleistung gedacht sind, im Sinne eines oder mehreres von elektrischem Leistungsverbrauch, Größe, Lärm und elektromagnetischer Emission konform zu sein.

4. System nach einem der vorstehenden Ansprüche, wobei die Abfallsterilisierungsvorrichtung (9) gestaltet ist, infektiösen medizinischen Abfall einem Trockensterilisierungsprozess zu unterziehen.

5. System nach einem der vorstehenden Ansprüche, wobei die Abfallsterilisierungsvorrichtung (9) gestaltet ist, infektiösen medizinischen Abfall einem widerstandsbasierten oder induktionsbasierten Wärmesterilisierungsprozess zu unterziehen.

6. System nach einem der vorstehenden Ansprüche, wobei der sterilisierte Abfallsammelbehälter (23) in einem Abteil (22) aufgenommen ist, das von dem getrennt ist, in dem der Abfallbehandlungstank (6) aufgenommen ist, und die sterilisierte Abfallentleerungsvorrichtung (11) gestaltet ist, sterilisierten Abfall durch Druckabbau von dem Abfallbehandlungstank (6) zu dem sterilisierten Abfallsammelbehälter (23) zu befördern.

7. System nach einem der vorstehenden Ansprüche, wobei die Abfallzerkleinerungs- und Mischvorrichtung (8) im Wesentlichen eine Klinge (16) umfasst, um Abfall zu zerkleinern und pulverisieren, der in dem Abfallbehandlungstank (6) aufgenommen ist, und von einem Motor (17) betrieben wird, der außerhalb des Abfallbehandlungstanks (6) eingerichtet ist.

8. System nach einem der vorstehenden Ansprüche, wobei jede Sammel- und Behandlungsvorrichtung (2) für infektiösen medizinischen Abfall auch eine Sterilisierungseffizienzbestätigungsvorrichtung (10) umfasst, die entnehmbar in dem Abfallbehandlungstank (6) aufgenommen ist und gestaltet ist, einen oder mehrere Sterilisierungsindikatoren (21) zu beherbergen, sodass sie denselben Bedingungen ausgesetzt werden, die in dem Abfallbehandlungstank (6) vorliegen.

9. System nach einem der vorstehenden Ansprüche, wobei jede Sammel- und Behandlungsvorrichtung (2) für infektiösen medizinischen Abfall weiter eine Abfallbehandlungstankdruckabbauvorrichtung (11) umfasst, die mit einer Luftfiltervorrichtung (27) bereitgestellt ist.

10. System nach einem der vorstehenden Ansprüche, wobei das Sensorsystem (32) konfiguriert ist, den Füllstand des sterilisierten Abfallsammelbehälters (23) zu messen und wobei die elektronische Steuereinheit (35) konfiguriert ist, die Notwendigkeit/Zweckmäßigkeit, den sterilisierten Abfallsammelbehälter (23) mit einem leeren zu ersetzen, zu ermitteln und an die Fernsteuerungszentrale (3) zu kommunizieren.

11. System nach einem der vorstehenden Ansprüche, weiter umfassend:
eine Fernsteuerungszentrale (3) in Kommunikation mit den Sammel- und Behandlungsvorrichtungen (2) für infektiösen medizinischen Abfall über ein Fernkommunikationssystem (4);
wobei die elektronischen Steuereinheiten (35) der Sammel- und Behandlungsvorrichtung (2) für infektiösen medizinischen Abfall konfiguriert sind, mit der Fernsteuerungszentrale (3) zu kommunizieren, um Daten dahin zu übertragen, die Betrieb der Sammel- und Behandlungsvorrichtungen (2) für infektiösen medizinischen Abfall anzeigen, und Befehle bezüglich des Betriebs der Sammel- und Behandlungsvorrichtungen (2) für infektiösen medizinischen Abfall davon zu empfangen und auszuführen;
wobei die Fernsteuerungszentrale (3) konfiguriert ist, Daten von den Sammel- und Behandlungsvorrichtungen (2) für infektiösen medizinischen Abfall zu empfangen und verarbeiten, und konfigurierbar den Betrieb davon basierend auf den spezifischen Bedürfnissen von Anwendungen und/oder Nutzern zu überwachen, steuern und melden.

12. System nach Anspruch 11, wobei die Fernsteuerungszentrale (3) auch konfiguriert ist, Befehle zu erzeugen und an die Sammel- und Behandlungsvorrichtungen (2) für infektiösen medizinischen Abfall zu senden, um Abfallbehandlungszyklen zu starten, basierend auf den spezifischen Bedürfnissen von Anwendungen und/oder Nutzern, wobei in Reaktion auf die Befehle die Sammel- und Behandlungsvorrichtungen (2) für infektiösen medizinischen Abfall konfiguriert sind, entsprechende Abfallbehandlungszyklen zu starten; die Fernsteuerungszentrale (3) auch konfiguriert ist, Abfallbehandlungszyklus-bezogene Daten von den Sammel- und Behandlungsvorrichtungen (2) für infektiösen medizinischen Abfall zu verarbeiten und erzeugerzugängliche Berichte über die durchgeführten Behandlungszyklen zu erzeugen.

13. System nach Anspruch 12, wobei die Fernsteuerungszentrale (3) auch konfiguriert ist, die Betriebsbedingungen der Sammel- und Behandlungsvorrichtungen (2) für infektiösen medizinischen Abfall zu überwachen und Verifizierung/Entleerung/Wartung/Ersatz der Sammel- und Behandlungsvorrichtungen (2) für infektiösen medizinischen Abfall vorherzusagen, deren Bedarf zu signalisieren und sie zu planen.

14. System nach Anspruch 12 oder 13, wobei die Fernsteuerungszentrale (3) auch konfiguriert ist, den Abfallerzeugern mit einem webbasierten oder mobilen anwendungsbasierten Dienst Zugang zu Informationen bezüglich der Abfallbehandlungszyklen bereitzustellen, die von deren Sammel- und Behandlungsvorrichtungen (2) für infektiösen medizinischen Abfall durchgeführt werden.

## Revendications

1. Système commandé à distance (1) pour le traitement *in-situ* de déchets médicaux infectieux produits par des petits producteurs de déchets, comprenant :
- des dispositifs de collecte et de traitement de déchets médicaux infectieux (2) destinés à être installés dans les installations de petits et moyens producteurs de déchets pour collecter et traiter des déchets médicaux infectieux et conçus pour être conformes avec l'utilisation des locaux destinés à des usages résidentiels, la pratique d'une profession ou la fourniture d'un service, tels que des locaux domestiques, des bureaux, des cabinets médicaux, dentaires et vétérinaires, des cliniques, ou similaires ;
dans lequel chaque dispositif de collecte et de traitement de déchets médicaux infectieux (2) comprend :
- un logement (5),
- un réservoir de traitement de déchets (6) logé dans le logement (5) et pourvu d'un dispositif d'entrée de déchets (7) pour des déchets médicaux infectieux,
- un dispositif de destruction et de mélange des déchets (8) logé dans le logement (5) et associé au réservoir de traitement des déchets (6) pour détruire et mélanger des déchets médicaux infectieux contenus dans celui-ci,
- un dispositif de stérilisation des déchets (9) logé dans le logement (5) et associé au réservoir de traitement des déchets (6) pour stériliser des déchets médicaux infectieux dans le réservoir de traitement de déchets (6),
- un conteneur de collecte des déchets stérilisés (23) logé de manière amovible dans le logement (5) pour collecter les déchets stérilisés,
- un dispositif de vidange des déchets stérilisés (11) logé dans le logement (5) pour transférer les déchets stérilisés du réservoir de traitement de déchets (6) vers le conteneur de collecte des déchets stérilisés (23),
- un système sensoriel (32) pour surveiller le fonctionnement du dispositif de collecte et de traitement des déchets médicaux infectieux (2) ; et
- une unité de commande électronique (35) connectée au système sensoriel (32), au dispositif de destruction et de mélange des déchets (8), au dispositif de stérilisation des déchets (9) et au dispositif de vidange des déchets (11), et configurée pour commander le fonctionnement du dispositif de collecte et de traitement des déchets médicaux infectieux (2) en :
∘ commençant les cycles de traitement des déchets en fonction des besoins des utilisateurs et/ou des demandes spécifiques,
∘ recevant et traitant les données relatives aux cycles de traitement des déchets et en générant des rapports accessibles par les producteurs concernant les cycles de traitements réalisés.

2. Système selon la revendication 1, dans lequel l'unité de commande électronique (35) est en outre configurée pour :
∘ exposer une interface utilisateur graphique pour permettre à un utilisateur de configurer et de diagnostiquer le dispositif de collecte et de traitement des déchets médicaux infectieux (2) via un dispositif électronique connectable de manière externe, et
∘ diagnostiquer le dispositif de collecte et de traitement des déchets médicaux infectieux (2) et stocker les données de diagnostiques accessibles via un dispositif électronique connectable de manière externe.

3. Système selon l'une quelconque des revendications précédentes, dans lequel les dispositifs de collecte et de traitement des déchets médicaux infectieux (2) sont conçus pour être conformes à l'utilisation des locaux destinés à des usages résidentiels, la pratique d'une profession ou la fourniture d'un service en termes de un ou plusieurs parmi la consommation d'énergie électrique, la taille, le bruit, et les émissions électromagnétiques.

4. Système selon l'une quelconque des revendications précédentes, dans lequel le dispositif de stérilisation des déchets (9) est conçu pour soumettre des déchets médicaux infectieux à un processus de stérilisation sèche.

5. Système selon l'une quelconque des revendications précédentes, dans lequel le dispositif de stérilisation des déchets (9) est conçu pour soumettre des déchets médicaux infectieux à un processus de stérilisation thermique à base d'induction ou à base de résistance.

6. Système selon l'une quelconque des revendications précédentes, dans lequel le conteneur de collecte des déchets stérilisés (23) est logé dans un compartiment (22) séparé de celui dans lequel le réservoir de traitement des déchets (6) est logé, et le dispositif de vidange des déchets stérilisés (11) est conçu pour transférer les déchets stérilisés du réservoir de traitement des déchets (6) vers le container de collecte des déchets stérilisés (23) par dépressurisation.

7. Système selon une quelconque des revendications précédentes, dans lequel le dispositif de destruction et de mélange des déchets (8) comprend essentiellement une lame (16) pour détruire et pulvériser les déchets, logée dans le réservoir de traitement des déchets (6) et pilotée par un moteur (17) agencé à l'extérieur du réservoir de traitement des déchets (6).

8. Système selon l'une quelconque des revendications précédentes, dans lequel chaque dispositif de collecte et de traitement des déchets médicaux infectieux (2) comprend également un dispositif de vérification d'efficacité de stérilisation (10) logé de manière amovible dans le réservoir de traitement des déchets (6) et conçu pour accueillir un ou plusieurs indicateurs de stérilisation (21) de sorte qu'ils sont soumis aux même conditions présentes dans le réservoir de traitement des déchets (6).

9. Système selon l'une quelconque des revendications précédentes, dans lequel chaque dispositif de collecte et de traitement des déchets médicaux infectieux (2) comprend en outre un dispositif de dépressurisation du réservoir de traitement des déchets (11) pourvu d'un dispositif de filtration d'air (27).

10. Système selon l'une quelconque des revendications précédentes, dans lequel le système sensoriel (32) est configuré pour mesurer le niveau de remplissage du conteneur de collecte des déchets stérilisés (23), et dans lequel l'unité de commande électronique (35) est configurée pour déterminer et communiquer à un centre de commande à distance (3) le besoin/l'opportunité de remplacer le conteneur de collecte des déchets stérilisés (23) par un conteneur vide.

11. Système selon l'une quelconque des revendications précédentes, comprenant en outre :
un centre de commande à distance (3) en communication avec les dispositifs de collecte et de traitement des déchets médicaux infectieux (2) via un système de communication à longue portée (4) ;
les unités de commande électroniques (35) du dispositif de collecte et de traitement des déchets médicaux infectieux (2) sont configurées pour communiquer avec le centre de commande à distance (3) pour transmettre à celui-ci des données indiquant le fonctionnement des dispositifs de collecte et de traitement des déchets médicaux infectieux (2) et pour recevoir et exécuter des ordres relatifs au fonctionnement des dispositifs de collecte et de traitement des déchets médicaux infectieux (2) ;
le centre de commande à distance (3) est configuré pour recevoir et traiter des données des dispositifs de collecte et de traitement des déchets médicaux infectieux (2) et pour surveiller, commander et établir des rapports de manière configurable sur ledit fonctionnement en fonction des demandes spécifiques et/ou des besoins des utilisateurs.

12. Système selon la revendication 11, dans lequel le centre de commande à distance (3) est aussi configuré pour générer et envoyer aux dispositifs de collecte et de traitement des déchets médicaux infectieux (2) des ordres pour commencer les cycles de traitement des déchets, en fonction des demandes spécifiques et/ou des besoins des utilisateurs, en réponse à quels ordres les dispositifs de collecte et de traitement des déchets médicaux infectieux (2) sont configurés pour commencer les cycles de traitement des déchets correspondants ; le centre de commande à distance (3) est aussi configuré pour traiter des données relatives aux cycles de traitement des déchets des dispositifs de collecte et de traitement des déchets médicaux infectieux (2) et pour générer des rapports accessibles par les producteurs concernant les cycles de traitement effectués.

13. Système selon la revendication 12, dans lequel le centre de commande à distance (3) est également configuré pour surveiller les conditions de fonctionnement des dispositifs de collecte et de traitement des déchets médicaux infectieux (2) et pour prédire, signaler le besoin, et planifier la vérification/la vidange/l'entretien/le remplacement des dispositifs de collecte et de traitement des déchets médicaux infectieux (2).

14. Système selon la revendication 12 ou 13, dans lequel le centre de commande à distance (3) est aussi configuré pour fournir aux producteurs de déchets un service d'accès basé sur une application mobile ou sur le Web des informations concernant les cycles de traitement des déchets effectués par leurs dispositifs de collecte et de traitement des déchets médicaux infectieux (2).
